# EUROPEAN PATENT SPECIFICATION

(11) **EP 4 133 095 B1**
(45) Date of publication and mention of the grant of the patent: **14.02.2024**
(21) Application number: 21722545.7
(22) Date of filing: 01.04.2021
(51) Int. Cl.: C12P 7/40, C12P 7/22

(54) **PROCESS FOR PREPARING PHENYLACETIC ACID**
VERFAHREN ZUR HERSTELLUNG VON PHENYLESSIGSÄURE
PROCÉDÉ DE PRÉPARATION D'ACIDE PHÉNYLACÉTIQUE

(30) Priority: 06.04.2020 IT 202000007279
(43) Date of publication of application: 15.02.2023
(73) Proprietor: Consiglio Nazionale Delle Ricerche, 00195 Roma (IT)
(72) Inventor: CASTAGNA, Antonio, 00195 Roma (IT); SERRA, Stefano, 00195 Roma (IT); VALENTINO, Mattia, 00195 Roma (IT)
(74) Representative: Robba, Pierpaolo
(86) International application number: PCT/IB2021/052744
(87) International publication number: WO 2021/205299

(56) References cited:
- EP-A2- 0 343 330
- WO-A1-2019/023019
- CN-B- 108 441 431
- US-A- 5 420 022
- BRAGA ADELAIDE ET AL: "Generation of Flavors and Fragrances Through Biotransformation and De Novo Synthesis", FOOD AND BIOPROCESS TECHNOLOGY ; AN INTERNATIONAL JOURNAL, SPRINGER-VERLAG, NEW YORK, vol. 11, no. 12, 19 September 2018 (2018-09-19), pages 2217-2228, XP036624171, ISSN: 1935-5130, DOI: 10.1007/S11947-018-2180-8 [retrieved on 2018-09-19]

## Description

### Technical Field

The present invention relates to a process for preparing phenylacetic acid.

More specifically, the present invention relates to a process for preparing natural phenylacetic acid from natural (L)-phenylalanine.

### Background Art

According to recent European regulations (European Parliament's Directive No. 1334/2008 of 16/12/2008; Article 3), flavouring agents (flavourings) can be defined as 'natural' only if they are derived from natural sources (by extraction) or they have been produced starting from natural precursors by using 'natural' transformation methods. Said directive further establishes what such methods may be. In particular, biotransformations fall within those methods that can be used for producing natural flavouring substances.

Phenylacetic acid is an important flavour characterized by a sweet, persistent odor recalling that of honey. Phenylacetic acid is naturally present and has been identified in plants as well as in cheeses and mushrooms. Having been recognized as safe for human health (FEMA-GRAS registered under No. 2878), it is used to flavour a large number of products, including beverages (both alcoholic and non-alcoholic ones), confectionery and bakery products, sausages and dairy products.

Phenylacetic acid cannot be obtained (except in traces) by extraction from natural sources. This results in a very high cost difference between the synthetic product (less than 50 euros/kg, as per Sigma-Aldrich price list) and the same product of natural origin (around 2550 euros/kg, as per Sigma-Aldrich price list).

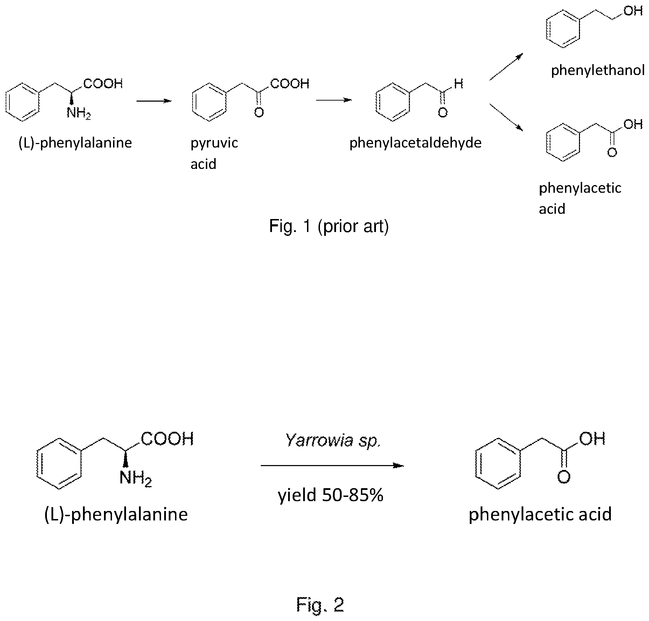

The methods used for the production of phenylacetic acid in natural form are essentially based on the microbiological/enzymatic degradation of natural phenylalanine, i.e. (L)-phenylalanine. According to the Ehrlich metabolic pathway, (L)-phenylalanine loses one ammonia molecule and is turned into phenylpyruvate (also known as phenylpyruvic acid), which in turn originates three different compounds of great importance in the flavouring field: phenylacetaldehyde, phenylethanol and phenylacetic acid (see Figure 1).

From an industrial standpoint, the major difficulty in producing phenylacetic acid lies precisely in the difficulty of directing the metabolic pathway exclusively towards the production of phenylacetic acid, to the detriment of the other products of the metabolic pathway.

It is known that most yeasts are able to transform phenylalanine into phenylethanol, and several processes for producing this alcohol are based on this degradation (Hua, D.; Xu, P. Recent advances in biotechnological production of 2-phenylethanol. Biotechnology Advances 2011, 29, 654-660).

It is also known that some microorganisms are able to convert phenylalanine directly to phenylacetic acid, but the processes based on this approach, described for example in patent publications JPS59232095A and US5420022A, give modest yields and/or use potentially pathogenic bacteria (e.g. *Pseudomonas* species).

Two-step processes have thus been developed, based on the transformation of phenylalanine into phenylethanol followed by oxidation of phenylethanol by acetic bacteria (Mihai, M.; Červeňanský, I.; Markoš, J. Production of phenylacetic acid from l-phenylalanine in dual reactor membrane hybrid system. Chemical Engineering and Processing: Process Intensification 2016, 110, 114-122). This class of microorganisms can effectively oxidize phenylethanol without growth inhibition by the substrate.

Another known biotechnological process (described in WO2018/217168A1 and in Zhang, L.H.; Liu, Q.; Pan, H.; Li, X.; Guo, D.Y. Metabolic engineering of escherichia coli to high efficient synthesis phenylacetic acid from phenylalanine. AMB Express 2017, 7, 7) is based on the use of genetically modified *E. coli.* In particular, *E. coli* is genetically modified to overexpress the enzymes styrene monooxygenase, styrene oxide isomerase and aldehyde dehydrogenase, and (L)-phenylalanine is converted to phenylacetic acid by a fermentation process using these enzymes.

Overall, to date, all the known processes mentioned above have at least one of the following problems/limitations:
1) they convert phenylalanine to phenylacetic acid with modest yields,
2) they use potentially pathogenic microorganisms, which are therefore not welcome by the food flavouring industry,
3) they are multi-step processes,
4) they use GMO microorganisms.

It is the object of the present invention to overcome the problems and limitations of prior art by providing a process for the preparation of natural phenylacetic acid that has high yields, is a single-step process and uses non-potentially pathogenic microorganisms.

A further object of the invention is to provide a process that does not use any GMO microorganisms.

These and other objects are achieved with the process for preparing phenylacetic acid as claimed in the appended claims.

### Summary of Invention

The process for preparing phenylacetic acid according to the invention essentially comprises the steps of:
- providing one or more strains of yeasts belonging to the genus *Yarrowia* and mutants thereof;
- providing a culture medium including phenylalanine;
- transforming phenylalanine into phenylacetic acid by fermentation of the strains of yeasts in the culture medium;
- isolating the phenylacetic acid from the broth of said fermentation.

The phenylalanine used in the process is preferably natural (L)-phenylalanine. Its initial concentration in the culture medium is preferably between 10 and 15 g/L.

The fermentation of the process according to the invention is a 'one pot' fermentation.

Advantageously, the fermentation of the process according to the invention is a single fermentation. In addition, the phenylalanine is transformed directly into phenylacetic acid, thus making the process simpler than those processes providing for the formation of intermediate products.

According to the invention, the fermentation is effected under aerobic conditions:
- in a flask, by using a volume of fermentation broth smaller than one fifth of the volume of the flask and stirring at a speed higher than 80 rpm, or
- in a fermenter, by using an airflow such that it ensures a oxygen partial pressure higher than 10% over the entire duration of fermentation.

In the case of fermentation in a fermenter, the aforesaid oxygen partial pressure is suitably guaranteed by means of an airflow greater than 0.2 v/v/min and by stirring at a speed higher than 100 rpm.

The molar yields of the transformation of (L)-phenylalanine into phenylacetic acid according to the process of the invention range from 50 to 85%, depending on the yeast strain used.

The molar yields of the transformation of racemic phenylalanine (DL-phenylalanine) into phenylacetic acid according to the process of the invention range from 25 to 80%, depending on the yeast strain used.

According to the invention, the yeast strains used belonging to the genus *Yarrowia* and mutants thereof are preferably non-genetically modified strains.

In addition, such strains preferably belong to the species *Yarrowia lipolytica* or *Yarrowia deformans* or *Yarrowia yakushimensis* or *Yarrowia bubula* and mutants thereof.

The microorganisms of the genus *Yarrowia* are regarded as safe for human health. Indeed, they belong to the biosafety level 1 (BSL 1). In addition, some of them are already used in processes for producing food flavourings. More particularly, strains of the species *Yarrowia lipolytica* are considered as safe for human health (Groenewald, M.; Boekhout, T.; Neuvéglise, C.; Gaillardin, C.; van Dijck, P.W.M.; Wyss, M. Yarrowia lipolytica: Safety assessment of an oleaginous yeast with a great industrial potential. Critical Reviews in Microbiology 2014, 40, 187-206), besides being currently employed in production processes for preparing food flavourings. For example, strains of *Yarrowia lipolytica* have obtained the GRAS (Generally Recognized as Safe) status for the production of erythritol (GRN 382), rebaudioside A (GRN 632), steviol glycosides of rebaudioside M (GRN 759).

Preferably, phenylacetic acid is isolated from the fermentation broth by means of the following steps:
- filtering and extracting the fermentation broth,
- drying and concentrating combined organic phases resulting from the preceding step of extraction of the fermentation broth,
- crystallizing a residue obtained from the previous drying and concentration of organic phases,
- distilling the crystallized residue.

The process according to the invention therefore makes it possible to produce natural phenylacetic acid in accordance with European Parliament Directive No. 1334/2008 of 16/12/2008, by virtue of the fact that a natural precursor such as (L)-phenylalanine is used, which is transformed by means of a biotransformation (fermentation) falling within the 'natural' transformation methods according to said directive.

### Brief Description of Drawings

These and other features and advantages of the present invention will become more apparent from the following description of preferred embodiments given by way of non-limiting examples with reference to the accompanying drawings, in which:
Figure 1 is a representation of the known Ehrlic metabolic pathway through which (L)-phenylalanine is transformed into phenylacetaldehyde, phenylethanol and phenylacetic acid;
Figure 2 is a representation of the direct transformation of (L)-phenylalanine into phenylacetic acid according to the invention.

### Description of Embodiments

A process for preparing phenylacetic acid from phenylalanine, and, in particular, natural phenylacetic acid from natural (L)-phenylalanine is described below. The process provides for a single fermentation of yeast strains belonging exclusively to the genus *Yarrowia* in a culture medium containing phenylalanine, preferably natural (L)-phenylalanine, at a concentration preferably lower than 20 g/L, more preferably between 10 and 15 g/L.

In particular, known strains belonging to the genus *Yarrowia* and mutants thereof, and preferably strains belonging to the species *Yarrowia lipolytica* and mutants thereof, are used. Preferably, instead, genetically modified microorganisms (GMO), even when belonging to the genus *Yarrowia,* are not used.

The fermentation of yeast strains according to the invention is a fermentation of the 'one pot' type and is conducted under aerobic conditions.

This fermentation enables the direct transformation of the amino acid (L)-phenylalanine into phenylacetic acid.

The conditions under which the process is carried out are the following:
- reaction temperature between 20 and 32 °C, more preferably between 26 and 30 °C;
- pH in a range between 5.5 and 7.5, more preferably of 6.5;
- fermentation duration between 4 and 10 days, more preferably 7 days.

In addition, the formation of phenylacetic acid by means of said fermentation is suitably obtained under conditions of efficient stirring and oxygenation. These conditions can be guaranteed in accordance with one of the following alternative experimental conditions:
a) Fermentation in a flask:
   Use of a volume of fermentation broth preferably smaller than one fifth of the flask volume and stirring at a speed preferably higher than 80 rpm.
b) Fermentation in a fermenter (bioreactor):
   Use of an airflow such that it ensures a oxygen partial pressure (pO₂) higher than 10% (relative to a maximum value of pO₂ that can be obtained with an airflow within the fermenter containing the culture medium before the addition of *Yarrowia*) over the entire duration of fermentation. This oxygen pressure is suitably guaranteed by means of an airflow greater than 0,2 v/v/min and by stirring at a speed higher than 100 rpm.

The molar yields of the transformation of (L)-phenylalanine into phenylacetic acid according to the process of the invention range from 50 to 85%, depending on the yeast strain used.

The molar yields of the transformation of racemic phenylalanine (DL-phenylalanine) into phenylacetic acid according to the process of the invention range from 25 to 80%, depending on the yeast strain used.

At the end of fermentation, the natural phenylacetic acid is conveniently isolated by means of extraction and distillation/crystallization methods.

The process of the invention is suitably conducted by using yeasts belonging to the genus *Yarrowia.*

According to the procedures described above, some illustrative and non-limiting examples of the process according to the invention are provided below.

### Example 1:

In a fermenter, there are loaded 2 L of a liquid culture medium having the following composition: yeast extract (3 g/L), malt extract (3 g/L), soybean peptone (5 g/L), glucose (10 g/L) and (L)-phenylalanine (12.5 g/L).

The fermenter is then sterilized at 121 °C for 15 minutes and the pH is adjusted at 6.50 by adding solutions of ammonia (10% w/v) or acetic acid (10% w/v).

The fermenter is subsequently stirred at 250 rpm at 28 °C and *Yarrowia lipolytica* (about 5 of wet yeast obtained by centrifugation of an active culture at about 3500 x g) suspended in about 10 mL of saline is then added thereto. In particular, the strain used in this example is *Yarrowia lipolytica* DSM 8218, belonging to the International Collection DSMZ (German Collection of Microorganisms and Cell Cultures GmbH).

A continuous airflow (about 1 L/min) is then introduced and fermentation is effected for 7 days, while continuing stirring the fermenter (at about 250 rpm).

The fermentation (biotransformation) is then stopped by adding an acid (for example, citric acid) until a pH of 4.0-4.5 is reached.

At this time, the fermentation broth is filtered through Celite and extracted with ethyl acetate (4 × 200 mL).

The combined organic phases resulting from the preceding extraction are then dried over anhydrous sodium sulfate and are subsequently concentrated under reduced pressure.

The residue (17.4 g) obtained from the preceding step crystallizes at room temperature and consists of quasi-pure phenylacetic acid.

Subsequent distillation of the residue under reduced pressure gives a yield of 15.0 g of phenylacetic acid (titrated at 97% by GC), molar yield 71%.

### Example 2:

In a one liter flask, there are loaded 100 mL of a liquid culture medium having the following composition: yeast extract (3 g/L), malt extract (3 g/L), soybean peptone (5 g/L), glucose (10 g/L) and (L)-phenylalanine (12.5 g/L).

The flask is closed with a cellulose plug and is then sterilized at 121 °C for 15 minutes. After cooling, an inoculum of *Yarrowia lipolytica* (about 0.5 g of wet yeast obtained by centrifugation of an active culture at about 3500 × g) suspended in about 5 mL of saline is added thereto. In this example, too, the strain used is *Yarrowia lipolytica* DSM 8218.

The flask is stirred at 150 rpm at 28 °C for 7 days.

The fermentation (biotransformation) is then stopped by adding an acid (for example, citric acid) until a pH of 4.0-4.5 is reached.

At this time, the fermentation broth is filtered through Celite and extracted with ethyl acetate (3 × 60 mL).

The combined organic phases resulting from the preceding extraction are then dried over anhydrous sodium sulfate and are subsequently concentrated under reduced pressure.

The residue (1.5 g) obtained from the preceding step is distilled under reduced pressure to give 0.72 g of phenylacetic acid (titrated at 96% by GC), molar yield 67%.

By way of illustrative and non-limiting example, further examples of fungal species of the genus *Yarrowia* that are used according to the present invention and the ID number of the relevant strain are provided in Table 1 here below. For each of these examples, the type of transformation process (in a fermenter or in a flask) as well as the corresponding molar yield of phenylacetic acid are indicated. All the experimental process conditions are the same as those described in Example 1 above (in case of fermentation in a fermenter) and in Example 2 above (in case of fermentation in a flask).

**Table 1:**

| Species | Strain | Type of fermentation | Yield |
|---|---|---|---|
| *Yarrowia lipolytica* | DSM 70562 | Fermenter | 75% |
| *Yarrowia deformans* | DSM 70561 | Fermenter | 72% |
| *Yarrowia yakushimensis* | CBS 10252 | Flask | 24% |
| *Yarrowia bubula* | CBS 12934 | Flask | 66% |

The strains identified with the code DSM belong, as mentioned above, to the Collection DSMZ (German Collection of Microorganisms and Cell Cultures GmbH), whereas those identified with the code CBS belong to the International Collection CBS Knaw (Westerdijk Fungal Biodiversity Institute).

## Claims

1. A process for preparing phenylacetic acid comprising the steps of:
- providing one or more yeast strains belonging to the genus *Yarrowia* and mutants thereof;
- providing a culture medium comprising phenylalanine;
- transforming phenylalanine into phenylacetic acid by fermentation of said one or more yeast strains in said culture medium, said phenylacetic acid being contained in a fermentation broth obtained by said fermentation;
- isolating the phenylacetic acid from said fermentation broth.

2. The process according to claim 1, wherein said phenylalanine is (L)-phenylalanine.

3. The process according to claim 1 or 2, wherein said fermentation is a 'one pot' fermentation.

4. The process according to any of the preceding claims, wherein said fermentation is a single fermentation and phenylalanine is transformed directly into phenylacetic acid.

5. The process according to any of the preceding claims, wherein the phenylalanine contained in said culture medium has a concentration between 10 and 15 g/L.

6. The process according to any of the preceding claims, wherein said fermentation is carried out:
- at a temperature between 20 and 32 °C;
- at a pH between 5.5 and 7.5;
- over a time period between 4 and 10 days.

7. The process according to any of the preceding claims, wherein said fermentation is carried out under aerobic conditions:
- in a flask, by using a volume of fermentation broth smaller than one fifth of the volume of the flask and by stirring at a speed higher than 80 rpm, or
- in a fermenter, by using an airflow such that it ensures an oxygen partial pressure higher than 10% over the entire duration of fermentation.

8. The process according to claim 7, wherein said fermentation in the fermenter is carried out by using an airflow greater than 0.2 v/v/min and by stirring at a speed higher than 100 rpm.

9. The process according to any of the preceding claims, wherein said yeast strains belonging to the genus *Yarrowia* and mutants thereof are non-genetically modified strains.

10. The process according to any of the preceding claims, wherein said yeast strains belonging to the genus *Yarrowia* are strains belonging to the species *Yarrowia lipolytica* and mutants thereof.

11. The process according to any of claims 1 to 9, wherein said yeast strains belonging to the genus *Yarrowia* are strains belonging to the species *Yarrowia deformans* or *Yarrowia yakushimensis* or *Yarrowia bubula* and mutants thereof.

## Patentansprüche

1. Verfahren zur Herstellung von Phenylacetat-Säure, umfassend die Schritte:
- Bereitstellen eines oder mehrerer Hefestämme, die zur Gattung *Yarrowia* und deren Mutanten gehören;
- Bereitstellen eines Kulturmediums umfassend Phenylalanin;
- Umwandeln von Phenylalanin in Phenylacetat-Säure durch Fermentation des einen oder der mehreren Hefestämme in dem Kulturmedium, wobei die Phenylacetat-Säure in einer durch die Fermentation erhaltenen Fermentationsbrühe enthalten ist;
- Isolieren der Phenylacetat-Säure aus der Fermentationsbrühe.

2. Verfahren nach Anspruch 1, wobei das Phenylalanin (L)-Phenylalanin ist.

3. Verfahren nach Anspruch 1 oder 2, wobei die Fermentation eine "Ein-Topf'-Fermentation ist.

4. Verfahren nach einem der vorhergehenden Ansprüche, wobei die Fermentation eine Einzelfermentation ist und Phenylalanin direkt in Phenylacetat-Säure umgewandelt wird.

5. Verfahren nach einem der vorhergehenden Ansprüche, wobei das im Kulturmedium enthaltene Phenylalanin eine Konzentration zwischen 10 und 15 g/L aufweist.

6. Verfahren nach einem der vorhergehenden Ansprüche, wobei die Fermentation durchgeführt wird:
- bei einer Temperatur zwischen 20 und 32 °C;
- bei einem pH-Wert zwischen 5,5 und 7,5;
- über eine Zeitspanne von 4 bis 10 Tagen.

7. Verfahren nach einem der vorhergehenden Ansprüche, wobei die Fermentation unter aeroben Bedingungen durchgeführt wird:
- in einem Kolben unter Verwendung eines Volumens an Fermentationsbrühe, das kleiner als ein Fünftel des Volumens des Kolbens ist, und durch Rühren mit einer Geschwindigkeit von mehr als 80 U/min, oder
- in einem Fermenter, indem ein Luftstrom verwendet wird, der einen Sauerstoffpartialdruck von mehr als 10 % über die gesamte Dauer der Fermentation gewährleistet.

8. Verfahren nach Anspruch 7, wobei die Fermentation in dem Fermenter unter Verwendung eines Luftstroms von mehr als 0,2 v/v/min und durch Rühren mit einer Geschwindigkeit von mehr als 100 U/min durchgeführt wird.

9. Verfahren nach einem der vorhergehenden Ansprüche, wobei die Hefestämme, die zur Gattung *Yarrowia* und deren Mutanten gehören, nicht-gentechnisch veränderte Stämme sind.

10. Verfahren nach einem der vorhergehenden Ansprüche, wobei die Hefestämme, die zur Gattung *Yarrowia* gehören, Stämme sind, die zur Art *Yarrowia lipolytica* und deren Mutanten gehören.

11. Verfahren nach einem der Ansprüche 1 bis 9, wobei die Hefestämme, die zur Gattung *Yarrowia* gehören, Stämme sind, welche zur Art *Yarrowia deformans* oder *Yarrowia yakushimensis* oder *Yarrowia bubula* und deren Mutanten gehören.

## Revendications

1. - Procédé de préparation d'acide phénylacétique comprenant les étapes suivantes :
- fournir une ou plusieurs souches de levure appartenant au genre *Yarrowia* et à ses mutants ;
- fournir un milieu de culture comprenant de la phénylalanine ;
- transformer la phénylalanine en acide phénylacétique par fermentation de ladite ou desdites souches de levure dans ledit milieu de culture, ledit acide phénylacétique étant contenu dans un bouillon de fermentation obtenu par ladite fermentation ;
- isoler l'acide phénylacétique à partir dudit bouillon de fermentation.

2. - Procédé selon la revendication 1, dans lequel ladite phénylalanine est la (L)-phénylalanine.

3. - Procédé selon l'une des revendications 1 ou 2, dans lequel ladite fermentation est une fermentation «monotope».

4. - Procédé selon l'une quelconque des revendications précédentes, dans lequel ladite fermentation est une fermentation unique et la phénylalanine est transformée directement en acide phénylacétique.

5. - Procédé selon l'une quelconque des revendications précédentes, dans lequel la phénylalanine contenue dans ledit milieu de culture a une concentration entre 10 et 15 g/L.

6. - Procédé selon l'une quelconque des revendications précédentes, dans lequel ladite fermentation est effectuée :
- à une température entre 20 et 32 °C ;
- à un pH entre 5,5 et 7,5 ;
- sur une période de temps entre 4 et 10 jours.

7. - Procédé selon l'une quelconque des revendications précédentes, dans lequel ladite fermentation est effectuée dans des conditions aérobies :
- dans un ballon, à l'aide d'un volume de bouillon de fermentation inférieur à un cinquième du volume du ballon et par agitation à une vitesse supérieure à 80 tpm, ou
- dans un fermenteur, à l'aide d'un écoulement d'air tel qu'il assure une pression partielle d'oxygène supérieure à 10% pendant toute la durée de la fermentation.

8. - Procédé selon la revendication 7, dans lequel ladite fermentation dans le fermenteur est réalisée à l'aide d'un écoulement d'air supérieur à 0,2 v/v/min et par agitation à une vitesse supérieure à 100 tpm.

9. - Procédé selon l'une quelconque des revendications précédentes, dans lequel lesdites souches de levure appartenant au genre *Yarrowia* et à ses mutants sont des souches non génétiquement modifiées.

10. - Procédé selon l'une quelconque des revendications précédentes, dans lequel lesdites souches de levure appartenant au genre *Yarrowia* sont des souches appartenant à l'espèce *Yarrowia lipolytica* et à ses mutants.

11. - Procédé selon l'une quelconque des revendications 1 à 9, dans lequel lesdites souches de levure appartenant au genre *Yarrowia* sont des souches appartenant à l'espèce *Yarrowia deformans* ou *Yarrowia yakushimensis* ou *Yarrowia bubula* et à leurs mutants.
